(19) **Europäisches Patentamt / European Patent Office / Office européen des brevets**

(11) **EP 4 635 487 A1**

(12) **EUROPEAN PATENT APPLICATION**
published in accordance with Art. 153(4) EPC

(43) Date of publication:
22.10.2025 Bulletin 2025/43

(21) Application number: 23904090.0

(22) Date of filing: 18.10.2023

(51) International Patent Classification (IPC):
A61K 31/07 (2006.01)     A61K 36/45 (2006.01)
A61K 31/201 (2006.01)     A61K 31/202 (2006.01)
A61P 27/02 (2006.01)

(52) Cooperative Patent Classification (CPC):
A61K 31/07; A61K 31/201; A61K 31/202;
A61K 36/45; A61P 27/02

(86) International application number:
PCT/MX2023/050062

(87) International publication number:
WO 2024/128905 (20.06.2024 Gazette 2024/25)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 13.12.2022 MX 2022016027

(71) Applicant: Centro de Retina Médica Y Quirúrgica,
S.C.
Zapopan, 45116 (MX)

(72) Inventors:
• SANTOS GARCÍA, Arturo
  Jalisco, 45116 (MX)
• ALTAMIRANO VALLEJO, Juan Carlos
  Jalisco, 45116 (MX)
• MORFÍN HERAS, Pedro
  Jalisco, 45116 (MX)

(74) Representative: Herrero & Asociados, S.L.
Cedaceros, 1
28014 Madrid (ES)

(54) **USES OF AN ORALLY ADMINISTERED FORMULATION CONTAINING ANTHOCYANINS AND PTEROSTILBENES FROM BLUEBERRY EXTRACT, OMEGA-3 FATTY ACIDS, LACTOFERRIN, VITAMINS A AND E AND EXCIPIENTS WITH SURFACTANT PROPERTIES, FOR PRIMARY TREATMENT AND/OR AS AN ADJUVANT FOR DRY EYE SYNDROME IN HUMAN BEINGS**

(57)     The invention relates to the use of a formulation containing anthocyanins and pterostilbenes from blueberry extract (*Vaccinium Myrtillus L*), omega-3 fatty acids, lactoferrin, vitamins A and E and excipients with surfactant properties, for primary treatment and/or as an adjuvant for dry eye syndrome in human beings.

In order to overcome the negative aspects of administering the formulation individually and to provide synergistic effects, the invention is characterized by the design of a formulation containing, among other components, polyphenols from blueberry extract, without eliminating the matrix, which promotes the bioactivity of the polyphenols present in same, as there is evidence that the presence of other polyphenols increases the bioavailability and bioactivity thereof. In addition, other elements of the matrix promote stability and absorption of polyphenols.

Fig. 3

EP 4 635 487 A1

**Description**

**TECHNICAL FIELD**

The present invention relates to the field of biotechnology and medicine, specifically to the area of ophthalmology. The invention comprises the use of an orally administered formulation that contains anthocyanins and pterostilbene derived from an extract of blueberry (*Vaccinium Myrtillus* L), omega-3 fatty acids, lactoferrin, vitamins A and E and excipients with surfactant properties for the primary treatment and/or as an adjuvant for dry eye syndrome in humans.

**BACKGROUND OF THE INVENTION**

Phenolic compounds are the largest natural group of non-energetic substances in the plant kingdom, with at least 10,000 structural variants of these molecules. Polyphenols are secondary metabolites of plants that contain aromatic rings with one or more hydroxyl groups and include flavonoids, phenolic acids and stilbenoids. Their possible effects on health are related to their notable antioxidant, anti-inflammatory, cardio-protective and neuro-protective biological activities. Plus, they are leading candidates to prevent or treat chronic diseases whose pathogenesis involves inflammation induced by oxidative stress.

In particular, the use of polyphenols in chronic eye diseases such as cataracts, macular degeneration and diabetic retinopathy could present strong potential benefits based on their inhibitory effects on oxidative stress, inflammation and angiogenesis pathways. There is evidence that phenolic compounds such as anthocyanins from blueberry extract are effective antioxidants in respect of the harmful effects of reactive oxygen species (ROS). In addition, pterostilbene, a component of blueberries, exhibits suppression of inflammation, apoptosis and oxidative stress.

Specifically, **dry eye disease** (DED), a chronic ocular surface disease, is characterized by significant overproduction of ROS, oxidative stress, and underlying inflammatory mechanisms with symptoms ranging from mild transient irritation to persistent dryness, burning, itching, pain and eye fatigue and visual disturbances. DED is a chronic, multifactorial ocular surface disease characterized by significant overproduction of ROS and oxidative stress leading to stress-induced inflammation.

Hyperosmolarity, a distinctive hallmark of DED, enhances the expression of proinflammatory cytokines, chemokines, and MMPs (matrix metalloproteinases). Among the MMPs that are overproduced during hyperosmolarity states of the ocular surfaces, there are significant amounts of MMP-2 (matrix metalloproteinase 2), MMP-3 (matrix metalloproteinase 3) and MMP-9 (Matrix metalloproteinase 9). On the other hand, ROS leads to cellular oxidative damage that also contributes to inflammation. Polyphenols inhibit oxidative stress pathways as they are effective antioxidants, and this reduces the harmful effects of ROS (Reactive Oxygen Species) by eliminating them.

Polyphenols are capable of inhibiting ocular inflammation by reducing the expression of the cytokines involved, for example, they downregulate TNF-$\alpha$, IL-1B, vascular endothelial growth factor (VEGF), intercellular adhesion molecule 1 (ICAM-1), vascular cell adhesion molecule 1 (VCAM-1) and nuclear factor (NF-kB). delphinidin 3,5-O-diglucoside is the most common anthocyanin found in blueberries and has been described as a potent suppressor of ROS formation in lacrimal gland tissue and an excellent conservant of tear secretion. Additionally, pterostilbene, a phenolic compound also found in abundance in blueberries, suppresses inflammation, apoptosis, and oxidative stress by blocking cell production and signaling pathways of the proinflammatory cytokines, such as TNF-$\alpha$ (tumor necrosis factor-$\alpha$), IL-1$\beta$ (interleukin 1 $\beta$), IL-4 (interleukin-4), MMP (most notably, MMP-9), COX-2 (cyclooxygenase 2), MAP kinases (mitogen-activated protein kinases) and phosphorylation of NF-kB p65 (nuclear factor that enhances kappa light chains of activated B cells).

Pterostilbene significantly reduces the overproduction of ROS induced by hyperosmolarity and restores the balance of oxygenase and antioxidant enzymes by suppressing the production of COX2 and at the same time improves the levels of SOD1 (superoxide dismutase 1) and PDRX4 (Peroxiredoxin-4).

Although several oral formulations containing anthocyanins and pterostilbene have been shown to provide potential benefits with respect to visual stress symptoms in patients with DED. However, these phenolic compounds are characterized by limited stability and solubility, very rapid metabolism and a very short half-life, resulting in very low bioavailability. However, there are multiple reports that describe the potential benefits of consuming polyphenols, particularly blueberry extracts, but they do not consider the above, so the clinical use of this type of products remains controversial.

Blueberries are part of a group of foods considered "superfoods" due to its content of natural compounds such as phenolic acids and esters, flavonoids, anthocyanins and procyanidins. Blueberry extract contains anti-inflammatory, antioxidant, and neuroprotective properties. Both anthocyanins and pterostilbene (PS) can significantly reduce the expression of anti-inflammatory mediators, tumor necrosis factor alpha (TNF-$\alpha$), interleukins (IL-1$\beta$, IL-6, MMP-2) and metalloproteinases (MMP-9). Likewise, they significantly reduce the levels of oxidative damage of biomarkers, malondialdehyde (MDA), 4-hydroxynonenal (4-HNE), aconitase-2 and 8-hydroxy 2'-deoxyguanosine (8-OHdG). Likewise, anthocyanins and PS restore homeostasis between the oxygenases and oxidative enzymes of the ocular surface by decreasing the expression of cyclooxygenase 2 (COX2) and restoring the antioxidant activity of enzymes such as superoxide dismutase 1 (SOD1) and peroxiredoxin 4 (PRDX4) in hyperosmotic stress states. Various publications suggest that oxidative stress states can be treated through the topical use of compounds to relieve discomfort associated with loss of tear film health. Some of them suggest that L-Carnitine and pterostilbene, present in blueberry extract, can reduce levels of oxidative stress in the tissues of the ocular surface.

On the other hand, many health professionals recommend that their patients incorporate supplements containing n-3 and n- 6 fatty acids (known as omega-3 and omega-6 fatty acids) into their daily diet since they are considered to provide anti-inflammatory activity and are not associated with substantial adverse events.

Omega-3 fatty acids, which were included in the target formulation of this invention, reduce acute and chronic inflammation. Dietary intake of omega-3 fatty acids decreases arachidonic acid mediators through competitive enzyme inhibition, shifting the balance toward a less inflammatory state. This anti-inflammatory effect is the reason for its effective use as an adjuvant in the treatment of DED. They have also been used successfully to alleviate dry eye symptoms and decrease tear evaporation rates in patients with dry eye related to computer vision syndrome. However, there is no scientific evidence strong enough for their systematic recommendation as a treatment to ameliorate symptoms or resolve signs associated with DED, despite its etiology.

Likewise, it is worth mentioning that the target formulation of this invention contains lactoferrin, a glycoprotein found in tears with anti-inflammatory, anti-microbial, anti-angiogenesis and antitumor activity. Low tear lactoferrin levels are associated with primary and secondary DED, and oral supplementation has been associated with improved tear film stability and improvement in DED symptoms. Since the study formulation was specially designed to improve the stability and bioavailability of polyphenols, one could speculate that the study formulation is effective due to the synergistic effect of the combination of polyphenols and omega-3 fatty acids.

Due to all of the above, the independent use of polyphenols contained in formulations of blueberry extract or n-3/n-6 fatty acids or lactoferrin for the treatment of DED is still debatable. However, there is scientific evidence that the administration of a formulation that combines polyphenols and omega-3 fatty acids offers significant synergistic effects that not only increase stability and the bioavailability of said nutraceuticals, but also the anti-inflammatory and antioxidant bioactivity. There is scientific evidence that the administration of a formulation that combines polyphenols and omega-3 fatty acids has a significant synergistic effect, not only in increasing the stability and bioavailability of said nutraceuticals, but also in the anti-inflammatory and antioxidant bioactivity of both. Specifically, a 300% increase in glutathione peroxidase activity, a 200% increase in plasma antioxidant capacity, a 50-100% decrease in lipid peroxidation carbonylation of proteins and urinary 8-isoprostanes, as well as a 50 to 200% attenuation in biomarkers of inflammation among other effects, compared to their individual capacities.

In the art previously described, there are multiple formulations designed as therapeutic agents for the treatment of dry eye disease. Said formulations may contain concentrations considered therapeutic of components such as essential fatty acids such as Omega-3, Omega-6, L-carnitine, vitamin C, vitamin E, copper and other minerals, as well as lactoferrin. However, a formulation designed as a dietary supplement has not been described, with clinical evidence containing anthocyanins and pterostilbene from blueberry extract in a synergistic combination with omega-3 fatty acids, lactoferrin and vitamins A and E and that can be used as a coadjuvant in the primary treatment and/or as a coadjuvant in dry eye syndrome in humans.

The invention disclosed in patent application WO2004006801: "Treatment for dry eye syndrome" with a publication date of January 22, 2004, deals with a therapeutic formulation to be used in the treatment of dry eye syndrome discloses the combination of Omega-3, Omega-6 in combination with vitamin E, lactoferrin and other nutrients for use in people with dry eye. This document discloses this combination, but it lacks components to mediate inflammation of the ocular surface such as those found in blueberry extract (anthocyanins and pterostilbene). The invention disclosed in patent application WO2004006801 lacks the significant synergistic effects that increase the stability and bioavailability of anthocyanins, pterostilbene and omega-3 fatty acids as it does not comprise blueberry polyphenols. Likewise, the formulation lacks synergy in its anti-inflammatory and antioxidant bioactivity as it does not have the aforementioned combination.

The invention disclosed in patent application MX/a/2017/016991 entitled "Formulation for the oral administration of blueberry extract as an auxiliary in the preservation of the health of the tear film in humans", has the application date of December 20, 2017 and was developed by the same creators of the invention that is the subject of this document. It discloses a formulation for oral administration in adults of blueberry extract (Vaccinium myrtillus L), lactoferrin, fish oil (eicosapentaenoic acid and docosahexaenoic acid), vitamin A and vitamin E is described. This formulation, which has antimicrobial properties, contains essential elements of the tear film and agents with the capacity to inhibit chronic inflammation of the ocular surface, has the potential to improve the health of the tear film by improving the secretion of the aqueous component of the tear film, increases the breakup time of the tear film, modulates the inflammatory process present in this condition and favors the preservation of the normal bacterial flora on the ocular surface. Likewise, this formulation presents significant synergistic effects of its polyphenols, by increasing the stability and bioavailability of anthocyanins, pterostilbene and omega-3 fatty acids and increasing the synergy of its anti-inflammatory and anti-oxidant bioactivity. The formulation in this invention is the main component in this new invention.

There is a technology for releasing drugs and food supplements known as soft gel capsules (Softgel caps), which are ideal containers for allopathic medications, phytotherapeutics and food supplements. This technology enables sufficient isolation for the adequate conservation of the active ingredients of a formulation, maintaining them with low levels of oxygen, avoiding internal reactions between the different active ingredients of the formulation and also facilitates the swallowing of the packaged compounds based on its non-stick properties and its presentation that is pleasant to the consumer.

Lastly, it is important to note that the use of a surfactant significantly improves the stability of the polyphenols, allowing the development of colloidal structures which resist light-, heat and alkaline conditions, preventing the degradation of polyphenols, preserving their structural integrity and enhances their resistance to oxidation. This becomes even more relevant when considering the use of a soft gelatin capsule for the target formulation of this invention, since the main site of anthocyanin absorption is the small intestine and not the stomach, as is commonly thought.

For all of the above, the invention developed comprises the use of an oral formulation that contains anthocyanins and pterostilbene derived from an extract of blueberry (Vaccinium Myrtillus L), omega 3 fatty acids, lactoferrin, vitamins A and E and excipients with surfactant properties for the primary treatment and/or as an adjuvant in the treatment of dry eye syndrome in humans.

The technical details of the developed invention are described below.

## OBJECT OF THE INVENTION

The object of this invention is to establish the uses and applications of an orally administered formulation for primary treatment and/or as an adjuvant for the treatment of dry eye syndrome in humans that contains anthocyanins and pterostilbene from blueberry extract (Vaccinium Myrtillus L), omega 3 fatty acids, lactoferrin, vitamins A and E and excipients with surfactant properties.

In order to overcome the negative aspects of the individual administration and to present the synergistic effects, the invention is characterized by the design of a formulation that comprises, among other compounds, polyphenols from extract of blueberry without eliminating the matrix, which favors the bioactivity of the polyphenols present in it, since there is evidence that the presence of other polyphenols increases the bioavailability and bioactivity of these. Additionally, other elements of the matrix with surfactant properties favor their stability and absorption.

## BRIEF DESCRIPTION OF THE FIGURES

Figure 1 represents the design of the tolerance and safety study.

Figure 2 represents the effectiveness study design.

Figure 3 represents the effectiveness study diagram.

Figure 4 represents the kinetics of release of total anthocyanins from the digested capsule in the stomach, duodenum and lower intestine.

Figure 5 represents the quantification of free anthocyanins once the stomach has been digested.

The capsule protects anthocyanins from digestion stomach and releases them in time for absorption in the duodenum and small intestine.

## DETAILED DESCRIPTION OF THE INVENTION

The characteristic details of the formulation that contains anthocyanins and pterostilbene from an extract of blueberry (Vaccinium Myrtillus L), omega 3 fatty acids, lactoferrin, vitamins A and E and excipients with surfactant properties for the primary and/or adjuvant treatment of dry eye syndrome in humans are clearly shown in the following description, which should serve as a reference for the tests carried out.

**The formulation comprises:**

*By combining the anthocyanins and pterostilbene of the blueberry extract with fish oil (omega-3 fatty acids), a*

*synergistic effect is obtained on the bioactivity of the micronutrients, and by adding a surfactant (PEG or chitosan) to the formulation, the formation of micelles or encapsulation of the polyphenols is promoted, which improves the stability and bioavailability of these and, consequently, their bioactivity.*

Based on the above, a formulation for oral administration in adults of a naturally occurring blueberry extract prepared for oral human consumption was generated that contains per 100 g:

- Blueberry extract (Vaccinium myrtillus L): 100 mg;
- Eicosapentaenoic acid (EPA): Equivalent to 9,000 to 12,000 mg;
- Docosahexaenoic acid (DHA): Equivalent to 6,000 to 8,000 mg;
- Lactoferrin that could be converted to polyethylene glycol (PEGylation) surfactant or associated with chitosan: Equivalent to 350 mg;
- Vitamin E: 15 mg (400 IU);
- Vitamin A: 1,500 IU;
- The excipient contains a surfactant agent.

Additionally, it is important to mention that the formulation is patent protected by the inventors of this new invention and the object of this new invention is to ameliorate symptoms associated with dry eye syndrome in humans.

*A clinical study to verify the application to dry eye syndrome of the formulation based on anthocyanins and pterostilbene from blueberry extract (Vaccinium Myrtillus L.), omega 3 fatty acids, lactoferrin, vitamins A and E and excipients with surfactant properties.*

Phase I-II clinical studies were carried out, in order to validate this formulation and its positive effects for the primary treatment and/or as an adjuvant in the treatment of dry eye syndrome in humans. The studies were prospective, randomized, double-blind, with control groups (placebos) involving four study groups [Dalia Ng, J.C. Altamirano-Vallejo, A. Gonzalez-De la Rosa, et al. 'An Oral Polyphenol Formulation to Modulate the Ocular Surface Inflammatory Process and to Improve the Symptomatology Associated with Dry Eye Disease']. Nutrients 14 (15), 3236].

The clinical study is described below:

The formulation used was the one previously described in this document and is characterized by comprising for every 100 g:

a) Blueberry extract (Vaccinium myrtillus L): 100 mg;
b) Eicosapentaenoic acid (EPA): Equivalent to 9,000 to 12,000 mg;
c) Docosahexaenoic acid (DHA): Equivalent to 6,000 to 8,000 mg;
d) Lactoferrin that could be added to Polyethylene glycol (PEGylation) surfactant or associated with chitosan: Equivalent to 350 mg;
e) Vitamin E: 15 mg (400 IU);
f) Vitamin A: 1,500 IU;
g) Excipient containing a surfactant.

The study was carried out in two parts. The first part (tolerability and safety study) was carried out on 20 healthy subjects, in a single group, prospective and blind study, where the tolerance and effectiveness of the formulation was evaluated. The second part of the clinical study (efficacy) was carried out in 104 eyes of 104 patients with a diagnosis of mild to moderate dry eye who satisfied all the inclusion and exclusion criteria of the study. (OSDI >13, Tear breakup time <10 seconds and MMP-9 positivity). The clinical study was carried out in a single center, in patients with a diagnosis of mild and moderate dry eye syndrome, in a private ophthalmology research unit with ISO 9001:20015 certification in the year 2021. The patients were divided into 4 different groups:

- Group administered the formulation as primary treatment (Group A).
- Groups administered the formulation as an adjuvant treatment to the administration of ocular lubricant (Groups A + L).
- Oral placebo administered group (Group P).
- Oral placebo administered group + ocular lubricant administered group (Groups P + L).

Approval clearance was obtained from the internal review board prior to patient enrollment (Ethics and Research Committee and Research Committee). This study adhered to the principles of the Helsinki Declaration. The protocol also adhered to the ICH guidelines on Good Clinical Practice and all other applicable local regulatory requirements and laws prior to enrollment, written informed consent was obtained from all subjects after a complete explanation of the nature and possible adverse events (AEs) of the study.

Subjects were over 18 years of age with a diagnosis of mild to moderate dry eye syndrome according to OSDI (Ocular Surface Disease Index), non-invasive tear film rupture time (NIF-BUT) <10 seconds and any degree of corneal staining with sodium fluorescein and conjunctival staining with lissamine green, with elevated levels of MMP-9 using the Quidel InflammaDry® kit, who suffered visual strain and screen work >8 hours/day.

**Intervention:**

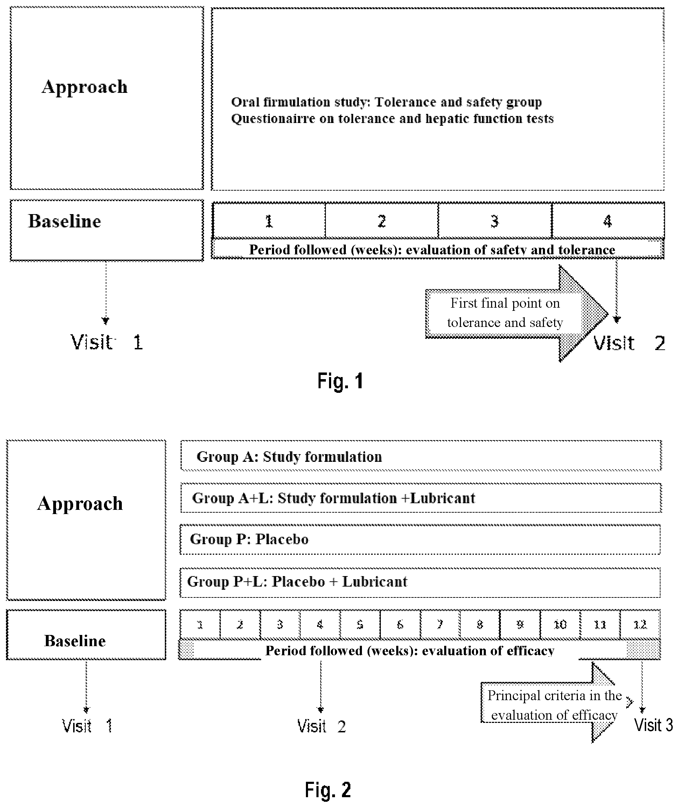

1. **Tolerability and safety study.** The design of the tolerability and safety study is shown in Figure 1. The 20 subjects in a single study group took 2 capsules every 24 hours for 4 weeks. Liver function tests were performed and a tolerance questionnaire (digestive tolerance) was completed by all patients.

2. **Efficacy study.** The design of the efficacy study is represented by Figure 2. Subjects were randomly assigned to one of four study groups that included the use as primary treatment of the study formulation (Group A) (The formulation described in this document), the groups using the study formulation (formulation described in this document) as an adjuvant treatment to the use of ocular lubricant (Groups A + L), the group using the orally administered placebo (Group P) and the groups using oral administration + the use of ocular lubricant (Groups P + L). Patients in groups A and P were instructed to take two capsules every 24 hours, while patients in groups L also added a drop of lubricant. (Commercially available lubricant indicated for the treatment of dry eye) twice a day for 12 weeks. During each of the visits, the subjects were subjected to different studies:

- Evaluation of symptomatology using the OSDI questionnaire (Ocular Surface Disease Index). This questionnaire is the most used by ophthalmologists to evaluate the symptoms of dry eye. If the score is >13, it is considered a positive criterion for the diagnosis of dry eye.
- Measurement of Best Corrected Visual Acuity (BCVA). The best-corrected visual acuity measurement was performed with the ETDRS (Early Treatment Diabetic Retinopathy Study) protocol, which is the most useful vision-measurement protocol utilized in clinical research.
- Complete clinical ophthalmological examination using a slit lamp. This test is non-invasive and is the test most used by ophthalmologists to assess the general condition of the ocular globe.
- Objective measurement of non-invasive tear breakup time (NIF-BUT) and average time (NIAvg -BUT) using the Schwind Sirius® topography equipment (CSO SRL, Italy).
- MMP-9 sampling with the Quidel equipment (InflammaDry®). This is the most specific test to objectively measure inflammation of the ocular surface. The test measures MMP-9, which is specific for inflammation of the ocular surface and is recognized as a determining factor in the chronic inflammation that occurs in dry eye. If dry eye treatment is effective, the subsequent test measurements should be negative.
- Impression cytology sampling. Through this study, a pathologist can assess the presence of inflammatory cells on the ocular surface (conjunctiva). If dry eye treatment is effective, the test for the presence of these cells in conjunctiva should result negative in subsequent measurements.
- Tear film osmolarity using the TearLab Osmolarity System® osmometer (TearLab, Escondido, CA, USA). Osmolarity is an important test to know the characteristics of the dry eye to be treated.
- Ocular surface stains with sodium fluorescein (AK-Fluor® Akorn, Lake Forest, IL, USA) and lissamine green (Rose Stone Enterprises, Alta Loma, CA, USA) to evaluate the surface of the cornea and conjunctiva. The OCT (Ocular Staining Score) scale of the SICCA (Sjögren Clinical Collaboration Alliance) was used. These stains are used by the ophthalmological community to evaluate the severity and response to treatment of dry eye syndrome.
- Schirmer test (Eagle Vision, Inc., Memphis TN, USA). This test is used by the ophthalmology community to evaluate the severity and response to treatment of dry eye syndrome.
- Intraocular pressure measurement (mmHg) (Icare® TA01i tonometer, Belleville, MI, USA). This test is part of the standard examination that every ophthalmologist performs on his patients.
- Evaluation of the fundus with indirect ophthalmoscopy (Killer Vantage Plus LED, Malvern, PA, USA). This test is part of the standard examination that every ophthalmologist performs on his patients.

To ensure the objectivity of these measurements, all were carried out at the same morning time (09:00am to 11:00am), in the same room, with silence, humidity control (40-50%) and the same controlled temperature (23 - 24$^0$C), without the use of reconditioned air during the review time and by the same researcher.

The patients' adherence-compliance to the use of the study medications (including the placebo) was evaluated with a written diary:

$$AD = (RA)100/IA$$

where AD is adherence, RA is the number of records of use of study medications and IA is the number of their administrations. An adherence value of <90% was considered as a failure in the adherence of said subjects to the treatment indicated in their study group. If this was the case, these subjects were excluded from the study.

## Results:

**1. Tolerability and safety study.** During the entire follow-up time, no adverse events were recorded. The product was well tolerated by all study subjects and liver function tests showed no significant changes at the end of the follow-up time. The study proved that the formulation is tolerable and safe for use in humans, the results are shown in the following table, which shows the demographic results of the security study.

| | A:Tolerability and Safety Group |
|---|---|
| Age | 36.4 $\pm$ 16.26 |
| Gender: | |
| Males (n) | 11 |
| Females (n) | 9 |
| Ocular findings | |
| Pseudophakia (n) | 2 |
| Basal BCVA (Early Treatment Diabetic Retinopathy Study (ETDRS) letters) | 83.2 $\pm$ 2.1 |
| | BCVA: Best corrected visual acuity |

Demographic characteristics and basal clinical values (Median $\pm$ SD). BCVA: Best corrected visual acuity; OSDI (Ocular surface disease index); NIF-BUT *(Non-invasive first break-up time)*

The following table shows the results of liver function tests. Liver function tests did not show significant changes after ingestion of the study formulation.

| Enzyme | Baseline | Week 4 | p Value |
|---|---|---|---|
| AST U/L | 21.46 $\pm$ 2.56 | 21.74 $\pm$ 1.9 | <0.05 |
| ALT U/L | 19.39 $\pm$ 4.2 | 17.73 $\pm$ 3.7 | <0.05 |
| GGT U/L | 15.86 $\pm$ 4.9 | 16.19 $\pm$ 4.6 | <0.05 |
| ALP U/L | 66.53 $\pm$ 13.4 | 66.08 $\pm$ 17.8 | <0.05 |
| LDH U/L | 234.76 $\pm$ 22.1 | 253.6 $\pm$ 19.6 | <0.05 |
| SA g/ dL | 4.26 $\pm$ 0.5 | 4.37 $\pm$ 0.4 | <0.05 |
| BIL mg/ dL | 0.35 $\pm$ 0.09 | 0.44 $\pm$ 0.05 | <0.05 |
| PT g/dL | 7.26 $\pm$ 0.6 | 7.62 $\pm$ 0.5 | <0.05 |

The hepatic function test comparing the baseline with week 4 of follow-up (Median $\pm$ SD) in the tolerability and safety group did not exhibit a significant difference (AST = aspartate transaminase, ALT = alanine transaminase, GGT = gamma-glutamyl transferase, ALT = alkaline phosphatase, LDH = L-lactis dehydrogenase, SA = serum albumin, BIL = bilirubin, PT = prothrombin time.)

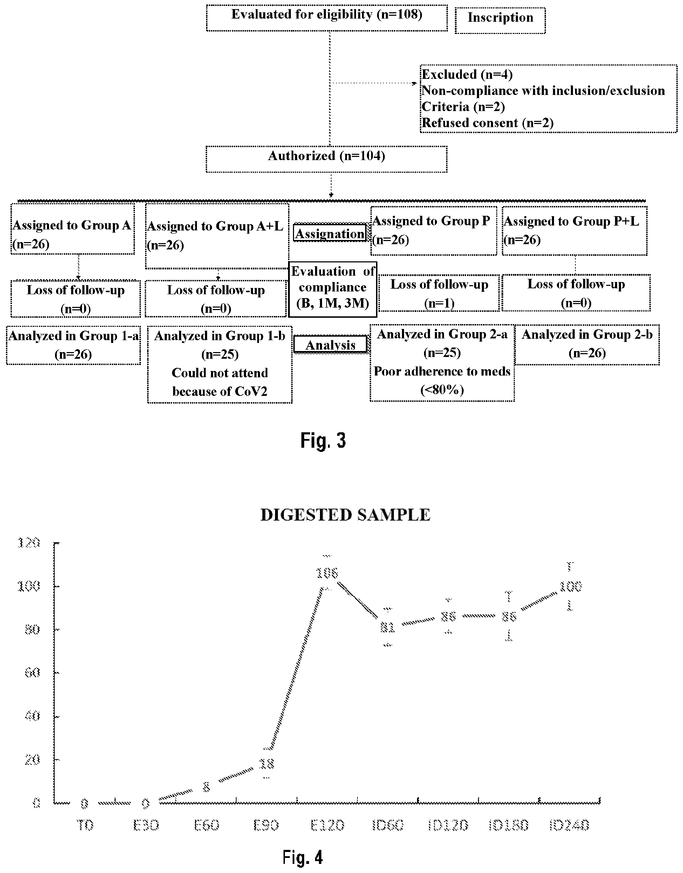

**2. Efficacy study.** The study included 104 patients with mild to moderate dry eye disease (DED) who met all inclusion criteria and none of exclusion criteria. Data from 102 subjects were analyzed (26 for group 1-a, 25 for group 1-b, 25 for group 2-a and 26 for group 2-b) since 2 did not complete the follow-up. The lack of compliance for those 2 patients was secondary to SARS CoV2 pandemic infection in one subject and poor medication adherence in another (<80%), Figure 3 is the diagram representing the enrolled subjects in the efficacy study, admitted, randomized and analyzed and the identification of the groups is as follows:

Groups:

- Group 1-a: Study formulation
- Group 1-b: Study formulation + Lubricant
- Group 2-a: Placebo
- Group 2-b: Placebo + Lubricant

Regarding the efficacy study, there was a statistically significant difference between the baseline, after 1 month and after 3 months in the OSDI (Ocular Surface Disease Index) test results in both treatment groups (group A and group A + L). Furthermore, both groups exhibited statistically significant differences between baseline and month 3 with respect to the non-invasive film break-up time (NIF-BUT) score and a positive trend related to the Schirmer test after 3 months. On the other hand, the average tear breakup time (NIAvg -BUT) also exhibited a positive trend of improvement with the treatment by the study formulation between baseline and the month of treatment compared to the baseline value in the A + L group. The P + L group exhibited a statistically significant difference in terms of the OSDI questionnaire between baseline and month 3. Regarding staining with lissamine green, the A + L group exhibited a statistical difference between the baseline and month 3 ($p = 0.0367$). The P + L group did not exhibit statistically significant differences in any of the remaining items. Of great relevance is that in groups A and A

+ L there was a significant difference in the inhibition of MMP-9 when 60% of the subjects were negative in the Quidel InflammaDry® test, showing the anti-inflammatory capacity of the formulation of this invention. Likewise, there was a significant difference in the improvement of the impression cytology of all the patients who took the study formulation (A and A + L), since none of the subjects who used the placebo (P and P + L) exhibited a statistically significant difference nor a positive trend in these specific items to measure ocular inflammation. Finally, the Placebo group did not exhibit any data with statistically significant differences. Consequently, our oral formulation as a primary treatment outperformed Placebo alone, and the oral polyphenol formulation used as an adjuvant to artificial tears was superior to the combination of Placebo and artificial tears.

Therefore, our data strongly suggest that this oral formulation of anthocyanins and pterostilbene from blueberry extract *(Vaccinium Myrtillus L),* omega 3 fatty acids, lactoferrin, vitamins A and E and excipients with surfactant properties exhibit efficacy for the primary treatment and/or as an adjuvant of dry eye syndrome in humans, by ameliorating ocular symptoms and improving the state of the tear film by having anti-inflammatory effects in patients with mild to moderate dry eye syndrome.

Furthermore, and in particular, none of the patients enrolled in this study presented gastrointestinal or ocular adverse effects. Therefore, this oral study formulation has the potential to be considered a primary and/or adjuvant treatment for patients suffering from visual fatigue resulting from dry eye syndrome. The following table presents the demographic and baseline data from the efficacy study (standard deviation).

The following table represents the demographic and baseline data of the efficacy study (Standard deviation). All patients presented diagnostic criteria for mild to moderate dry eye syndrome.

| | A (Formulation) | A+L (Formulation + Lubricant) | P (Placebo) | P+L (Placebo + Lubricant) |
|---|---|---|---|---|
| Age | 40.9 ± 10.46 | 41.5 ± 6.65 | 39.9 ± 8.35 | 40.9 ± 7.4 |
| Gender: | | | | |
| Males (n) | 13 | 10 | 10 | 12 |
| Females (n) | 13 | 15 | 15 | 14 |
| Hypertension (n) | 5 | 2 | | |
| Ocular findings Pseudophakia (n) | 2 | 4 | 3 | 3 |
| Basal BCVA (ETDRS) letters) | 82.5 ± 13.2 | 82.1 ± 1.2 | 83.1 ± 1.1 | 82.2 ± 1 |
| OSDI (Score) | 21.08 ± 5.28 | 21.38 ± 5.82 | 21.58 ± 6.38 | 21.53 ± 5.42 |
| Osmolarity | 286.61 ± 18.72 | 285.44 ± 14.42 | 282.42 ± 13.93 | 284.71 ± 18.11 |
| NIF-BUT (s) | 8.37 ± 5.86 | 9.52 ± 6.28 | 9.33 ± 6.10 | 9.24 ± 5.24 |
| NIAvg-BUT (s) | 11.06 ± 4.47 | 10.25 ± 3.45 | 11.44 ± 3.45 | 11.89 ± 4.95 |
| Schirmer test (mm) | 21.84 ± 9 | 21.53 ± 8 | 24.88 ± 11.09 | 24.06 ± 9.4 |

The following table represents an analysis of quantitative variables in subjects with dry eye treated with the formulation of this invention and the placebo. Subjects in the study group of the formulation described in this document exhibited statistically significant changes in OSDI and tear breakup time (NIF-BUT). The Schirmer test and the average tear breakup time exhibited a positive trend in the study group of our invention. None of the subjects in the placebo group exhibited significant variation in any of the items.

|  | A | | | P | | |
|---|---|---|---|---|---|---|
| Variable /Visits | B | 1 | 3 | B | 1 | 3 |
| OSDI Score | 21.08 ± 5.28 | 13.88 ± 7.21* | 9.73 ± 6.84* | 21.58 ± 6.38 | 16.23 ± 8.95 | 17.07 ± 12.19 |
| Osmolarity | 86.61 ± 18.72 | NA | 286.07 ± 11.94 | 282.42 ± 13.95 | NA | 276.07 ± 15.84 |
| NIF-BUT(s) | 8.37 ± 5.86 | 8. 47 ± 5.12 | 10.94 ± 5.45* | 9.33 ± 6.10 | 8.21 ± 5.87 | 7.83 ± 3.01 |
| NIAvg-BUT(s) | 11.06 ± 4.47 | 10.16 ± 4.66 | 12.25 ± 4.23 | 11.44 ± 4.55 | 10.03 ± 5.09 | 9.52 ± 4.58 |
| Schirmer test (mm) | 21.84 ± 9 | 19.15 ± 11.37 | 24.84 ± 8.78 | 24.08 ± 11.09 | 17.57 ± 9.74 | 19.03 ± 10.81 |

**B: Baseline; A: Study oral formulation; NA: Not Applicable; P: Placebo; OSDI (Ocular surface disease index); NIF-BUT (Non-invasive first break-up time); NIAve-BUT: Average time for non-invasive tear breakup. *Statistically significant differences from baseline**

The following table shows the analysis of quantitative variables of ocular surface inflammation lar (MMP-9). Subjects in the study formulation group (innovation described in this document) exhibited a statistically significant reduction in the objective measurement of MMP-9, which is the specific marker of inflammation of ocular surface. The anti-inflammatory activity of the formulation of our study is demonstrated by this data. None of the subjects included in the placebo group exhibited negativity in this test, which is proof that the placebo group does not exhibit anti-inflammatory activity.

| MMP-9 Score/visit | Baseline | Week 3 |
|---|---|---|
| Group 1 | 10 | 3* |

**MMP-9: Matrix metalloproteinase 9**
**\*Significant clinical difference**

The following table shows the analysis of quantitative variables in the subjects with mild to moderate dry eye syndrome in the group of combination of study formulation and lubricant drops (A + L) and in the placebo + lubricant drops group (P + L). The A + L group showed statistically significant improvement between the baseline measurement and month 3 in the OSDI, NIF-BUT and NIAvg-BUT areas. Additionally, the Schirmer test exhibited a positive trend. The P + L group exhibited a statistically significant difference only in OSDI and in NIF-BUT.

|  | A+L | | | P+L | | |
|---|---|---|---|---|---|---|
| Variable/Visits | B | 1 | 3 | B | 1 | 3 |
| OSDI Score | 21.38 ± 7.5 | 13.50 ± 8.66* | 9.15 ± 8.42* | 21.54 ± 6.13 | 14.42 ± 6.65* | 15 ± 8.75^ |
| Osmolarity | 283.84 ± 16.9 | NA | 283 ± 6.72 | 286 ± 13.69 | NA | 279 ± 13.8 |
| NIF-BUT (s) | 7.49 ± 5.74 | 6.26 ± 5.43* | 11.85 ± 5.8* | 9.9 ± 5.66 | 9.33 ± 6.03 | 8.59 ± 5.73 |
| NIAvg-BUT (s) | 8.67 ± 5.16 | 8.64 ± 4.68 | 12.61 ± 5.01* | 11.37 ± 4.92 | 10.49 ± 5 | 10.25 ± 4.65 |
| Schirmer test (mm) | 20.96 ± 10.45 | 18.88 ± 9.59 | 21.57 ± 9.5 | 24.8 ± 18.65 | 21 ± 10.5 | 23.76 ± 10.19 |

**B: Baseline; A: Study oral formulation; NA: Not Applicable; P: Placebo; OSDI (Ocular surface disease index); NIF-BUT (Non-invasive first break-up time); NIAve-BUT: Average time for non-invasive tear breakup.**

The following table represents the quantitative analysis of ocular surface staining. In relation to the ocular surface stains, the A + L group exhibited a statistically significant difference, something which is very relevant, since this marker is specific to chronic damage to the ocular surface caused by dry eye. The P + L group did not exhibit a statistically significant improvement in corneal stains, which are an indicator of damage to the ocular surface in dry eye.

| Grade/Visits n | A+L | | | P+L | | | p | | |
|---|---|---|---|---|---|---|---|---|---|
| | B | 1 | 3 | B | 1 | 3 | B | 1 | 3 |
| 0 | 3 | 8 | 19 | 1 | 9 | 9 | 0.693 | 0.7074 | 0.0367* |
| I | 12 | 11 | 4 | 11 | 11 | 8 | | | |
| II | 6 | 7 | 3 | 7 | 5 | 7 | | | |
| III | 5 | 0 | 0 | 7 | 1 | 2 | | | |
| IV | 0 | 0 | 0 | 0 | 0 | 0 | | | |
| V | | | | | | | | | |

B: Baseline; A: Study oral formulation; 1 weekly visit 4, 3, weekly visit 12; Study oral formulation; P: Placebo; L: Lubricant. *Statistically significant differences from baseline

### *Justification of the invention*

Dry eye syndrome is one of the most commonly observed clinical conditions worldwide, with an estimated population prevalence of 9% to 30%. However, several scientific reports show that the extensive use of computers and monitors, especially since the start of the SARS-CoV-2 pandemic, has amplified the impact of dry eye related visual fatigue by up to 70.8% in medical students. In addition to increased screen exposure, the use of face masks has been associated with an exacerbation of DED symptoms by 26.09% and there has been an 18.3% increase in the number of dry eyes associated with masks.

Nowadays, the instillation of artificial tears has become the therapeutic approach to DED. However, treatment compliance is as low as 10.2%, since 6 out of 10 patients use their drops only when necessary to relieve subjective symptoms. Additionally, most patients report that the instillation of eye drops alone does not provide sufficient relief, leading them to seek home remedies in up to 76% of cases, which generates frustration, particularly since dry eyes are perceived as a "elderly illness" or something not as serious as allergies. Therefore, there is a need for various therapeutic approaches to prevent poor compliance, alleviate DED symptoms, and improve patient comfort.

An extraordinary option is the oral route, however, today there are no orally administrated products that have been demonstrated to be effective in the treatment of dry eye. Currently, there is no formulation for oral administration that contains polyphenols and bioactive substances from blueberry extract in combination with the use of n-3 and n-6 fatty acids (known as omega-3 and omega-6 fatty acids), PEGylated lactoferrin and vitamins A and E that have proven their clinical effectiveness in dry eye syndrome for use as a primary treatment or as an adjuvant to lubricants. This is the first time that these unexpected and effective findings in the treatment of dry eye using an orally administered product with this combination of elements have been described.

### *Impact of the invention.*

Dry eye syndrome is one of the most commonly observed clinical conditions worldwide, with an estimated population prevalence of 9% to 30%. However, several scientific reports show that the extensive use of computers and monitors, especially since the start of the SARS-CoV-2 pandemic, has amplified the impact of dry eye related visual fatigue by up to 70.8% in medical students. In addition to increased screen exposure, the use of face masks has been associated with an exacerbation of DED symptoms by 26.09% and there has been an 18.3% increase in the number of dry eyes associated with masks.

This makes the field of ophthalmic therapeutics particularly interesting for researchers and entrepreneurs with a scientific-technological base; Given that more than half of the population is at risk of dry eye, the impact of this invention could benefit millions of men and women computer and monitor users who suffer from dry eye.

### **Kinetic tests to assess the release of active ingredients from a soft capsule.**

Kinetic digestion tests were carried out to assess the release of active ingredients from a soft capsule with the following characteristics:

**Objective:** Follow the process of release of anthocyanins from a capsule containing blueberry extract rich in polyphenols during the ex vivo digestion process.

**Method used:**

Capsule Digestion Simulator of the stomach and small intestine.

**Methodology.**

Digestion of capsules in the stomach and small intestine.

For the digestion of the capsules in stomach and small intestine conditions, the ARIS system (Automatic and Robotic Intestinal System) was used with the conditions described by (García-Gamboa et al ., 2020) and following the patented procedures described (González Avila et al ., 2017) under fasting conditions by digesting two capsules in a volume of 100 ml.

Sample collection was performed every 30 minutes in the stomach section and every hour in the small intestine section. Each measurement was performed in quintuple and the average of the five measurements and the standard deviation in each case are reported.

**Quantification of anthocyanins in samples collected during the digestion process.**

The quantification of anthocyanins was carried out following the pH differential spectrophotometric method reported by the AOAC in 2005. Each sample was diluted in both potassium chloride buffer (pH 1) and buffer of sodium acetate (pH4.5) and its absorbance was measured at 520 and 700 nm. Anthocyanin concentrations are expressed as equivalents of cyanidin-3-glucoside in mg/L (eqC3G) using the following equation:

$$eq\,C3G = \frac{[(A_{500} - A_{700})pH_1 - (A_{500} - A_{700})pH_{4.5}][PM\,C3G][FD][1000]}{lx\varepsilon}$$

Where: $A_{500}$ and $A_{700}$= the absorbances at these wavelengths, PM C3G = molecular weight of cyanidin-3-glucoside, FD = dilution factor, l= 1cm, $\varepsilon$= 26900 L/mol cm.

**Results.**

Figure 4 represents the evolution of the concentration of anthocyanins, determined as described above, over time and under the conditions of pH and presence of enzymes in the stomach (30, 60, 90 and 120 min) and small intestine (60, 120, 180 and 240 min). In this figure the kinetics of anthocyanin release through the digestion process, where T0 represents the initial time, EX represents the stomach sampling times while IDX represents the sample in the small intestine. The concentration is expressed in mg/L.

**During the digestion process, in the conditions of the stomach, we can observe that the release of anthocyanins is low and slow for the first 90 minutes, at minute 120 the release shoots up and in the small intestine the anthocyanins begin to be absorbed or degraded, in a sustained manner.**

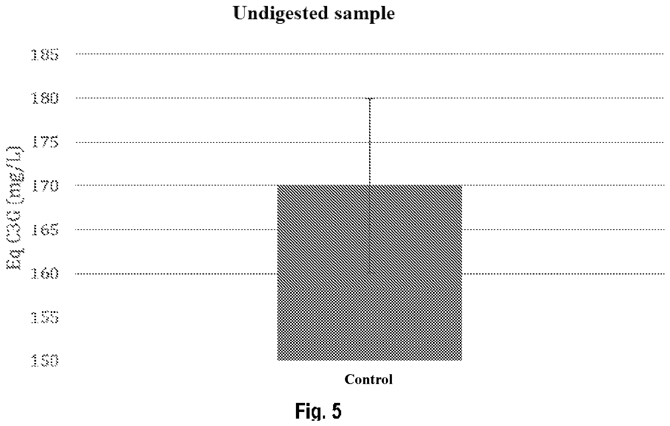

In parallel, the dilution of a capsule was carried out only in water, without changing the pH or other conditions, this solution was maintained for the same time as the experiment and in the end, at 240 min, a determination of said anthocyanins was performed, obtaining a concentration of 170 $\pm$ 10 mg/L (this is represented in Figure 5, which represents the total concentration of anthocyanins of the control sample that consists of the capsule dissolved in water for 240 min), in aqueous blueberry extracts in the laboratory there is an average of 220 mg/L of total anthocyanins which demonstrates that the capsule contains a concentration of anthocyanins of the expected theoretical order of magnitude according to their content in extracts obtained in the laboratory (-22%).

*In conclusion, the capsules of the study formulation protects the blueberry anthocyanins from stomach digestion so as to be subsequently released for absorption in the duodenum and small intestine, which are the anatomical areas of greatest absorption thereof.*

*Verified formulation for oral administration that contains anthocyanins and pterostilbene from blueberry extract (Vaccinium Myrtillus L), omega-3 fatty acids, lactoferrin, vitamins A and E and excipients with surfactant properties for the primary treatment and/or as an adjuvant of dry eye syndrome in humans.*

Regarding the verification presented in the previous study, the application of the formulation that is claimed as the invention is as follows:

An oral administration formulation that contains anthocyanins and pterostilbene from an extract of blueberries (*Vaccinium Myrtillus L*), for use in the primary treatment and/or as an adjuvant of dry eye syndrome in humans contains per 100 g:

a) Blueberry extract (Vaccinium Myrtillus L): 100 mg;

b) Eicosapentaenoic acid (EPA) equivalent to 9000 to 12,000 mg;

c) Docosahexaenoic acid (DHA) equivalent to 6000 to 8,000 mg;

d) Lactoferrin that can bind to polyethylene glycol (PEGylation) surfactant or associated with chitosan equivalent to 350 mg;

e) Vitamin E: 15 mg (400IU);
f) Vitamin A: 1500IU;
g) An excipient which contains a surfactant.

Additionally, the previously described formulation has the following characteristics to be used in:

- Obtaining a synergistic effect associated with the combination of anthocyanins and pterostilbene from blueberry extract with omega-3 fatty acids for primary treatment or as an adjuvant in dry eye syndrome in humans;

- An increase in the stability and bioavailability of the compounds contained therein in humans with dry eye syndrome;

- In a soft capsule for the release of anthocyanins in the duodenum and small intestine, which contains cranberry extract rich in polyphenols and protects them during the stomach digestion process;

- Increased tear film stability in humans with dry eye syndrome;

- Decreased evaporation of the tear film of humans with dry eye syndrome;

- An increase in tear breakup time in humans with dry eye syndrome;

- Blocking the nuclear transcription factor NFkB and inhibiting the expression of inflammatory interleukins and matrix metalloproteinases on the ocular surface in humans with dry eye syndrome;

- The decrease in inflammation of the ocular surface of humans with dry eye syndrome;

- Decreased conjunctival and/or corneal de epithelialization in humans with dry eye syndrome;

- The improvement of the symptoms associated with dry eye syndrome in humans.

The foregoing description of the definitions disclosed is provided to allow any person skilled in the art to make or use the present invention. Various modifications to these definitions and/or implementations will be readily apparent to those skilled in the art, and the generic principles defined herein may be applied to other embodiments without departing from the spirit or scope of the invention. Thus, the present invention is not intended to be limited to the embodiments disclosed herein, but rather should be granted the broadest scope consistent with the following claims and novel principles and features described in this document.

## Claims

1.  An orally administered formulation which contains anthocyanins and pterostilbene from extracts of blueberries (*Vaccinium Myrtillus L*) for use in the primary treatment and/or as a coadjuvant in respect of dry eye syndrome in human beings which contains per 100g:

    h) Blueberry extract (*Vaccinium Myrtillus L*)*:* 100 mg;
    i) Eicosapentaenoic acid (EPA) equivalent to 9000 to 12,000 mg;
    j) Docosahexaenoic acid (DHA) equivalent to 6000 to 8000 mg;
    k) Lactoferrin that can bind to polyethylene glycol (PEGylation) surfactant or associated with chitosan equivalent to 350 mg;
    l) Vitamin E: 15 mg (400 IU);
    m) Vitamin A: 1500 IU;
    n) Excipient which comprises a surfactant.

2.  The orally administered formulation according to claim 1 for use in obtaining a synergistic effect associated with the combination of anthocyanins and pterostilbene extracted from blueberries with omega-3 fatty acids when used in the primary treatment and/or as a coadjuvant in respect of dry eye syndrome in human beings

3.  The orally administered formulation according to claim 1 for use in the augmentation of the stability and bioavailability of the compounds contained therein in humans with dry eye syndrome.

4. The orally administered formulation according to claim 1 for use in combination with excipients with surfactant properties in a soft gelatine capsule so as to protect the bioactive contents thereof during the stomach digestive process and to liberate them in the small intestine.

5. The orally administered formulation according to claim 1 for use in the increase of the tear film stability in humans with dry eye syndrome.

6. The orally administered formulation according to claim 1 for use in the reduction of the evaporation of the tear film in humans with dry eye syndrome.

7. The orally administered formulation according to claim 1 for use in increasing the tear film rupture time in humans with dry eye syndrome.

8. The orally administered formulation according to claim 1 for use in blocking the nuclear transcription factor NFkB and inhibiting the expression of inflammatory interleukins and matrix metalloproteinases on the ocular globe surface in humans with dry eye syndrome

9. The orally administered formulation according to claim 1 for use in the reduction of the inflammation of the ocular surface in humans with dry eye syndrome.

10. The orally administered formulation according to claim 1 for use in decreasing conjunctival and/or corneal de epithelialization in humans with dry eye syndrome.

11. The orally administered formulation according to claim 1 for use in the amelioration of the symptoms associated with the inflammatory process in dry eye syndrome in humans.

EP 4 635 487 A1

Fig. 1

Fig. 2

Evaluated for eligibility (n=108)    Inscription

Excluded (n=4)
Non-compliance with inclusion/exclusion
Criteria (n=2)
Refused consent (n=2)

Authorized (n=104)

| Assigned to Group A (n=26) | Assigned to Group A+L (n=26) | Assignation | Assigned to Group P (n=26) | Assigned to Group P+L (n=26) |

| Loss of follow-up (n=0) | Loss of follow-up (n=0) | Evaluation of compliance (B, 1M, 3M) | Loss of follow-up (n=1) | Loss of follow-up (n=0) |

| Analyzed in Group 1-a (n=26) | Analyzed in Group 1-b (n=25) Could not attend because of CoV2 | Analysis | Analyzed in Group 2-a (n=25) Poor adherence to meds (<80%) | Analyzed in Group 2-b (n=26) |

**Fig. 3**

### DIGESTED SAMPLE

**Fig. 4**

## Undigested sample

Fig. 5

<table>
<tr><td colspan="2"><strong>INTERNATIONAL SEARCH REPORT</strong></td><td>International application No.<br>PCT/MX2023/050062</td></tr>
</table>

### A. CLASSIFICATION OF SUBJECT MATTER

See extra sheet

According to International Patent Classification (IPC) or to both national classification and IPC

### B. FIELDS SEARCHED

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, INTERNET

### C. DOCUMENTS CONSIDERED TO BE RELEVANT

| Category* | Citation of document, with indication, where appropriate, of the relevant passages | Relevant to claim No. |
|---|---|---|
| X | DALIA, NG. et al . An Oral Polyphenol Formulation to Modulate the Ocular Surface Inflamatory Process and to Improve the Symptomatology Associated with Dry Eye Disorder.. Nutrients 2022, 07/08/2022, Vol. 14, N° 3236, pages 1-15 Retrieved from <URL: http://doi.org/10.3390/nu14153236> Abstract; paragraphs 2.3 and 5;  table 1. | 1-11 |
| X | WO 2019125127 A1 (CENTRO DE RETINA MÉDICA Y QUIRÚRGICA) 27/06/2019, Abstract, page 13, lines 11-26; page 19, lines 1-12, 22-23; page 20, lines 5-6; claims 1-4 | 1-11 |

☐ Further documents are listed in the continuation of Box C.    ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 21/11/2023 | **(23/11/2023)** |

| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>J. López Nieto<br><br><br>Telephone No. 913498426 |
|---|---|

Form PCT/ISA/210 (second sheet) (July 2022)

## INTERNATIONAL SEARCH REPORT

Information on patent family members

International application No.

PCT/MX2023/050062

| Patent document cited in the search report | Publication date | Patent family member(s) | Publication date |
|---|---|---|---|
| WO2019125127 A1 | 27.06.2019 | US2021076726 A1 | 18.03.2021 |
| | | JP2021508502 A | 11.03.2021 |
| | | CA3102251 A1 | 27.06.2019 |
| | | BR112020012073 A2 | 24.11.2020 |
| | | | 25.09.2020 |
| | | CN111712237 A | 21.06.2019 |
| | | MX2017016991 A | 28.10.2020 |
| | | EP3730130 A1 | 15.09.2021 |
| | | EP3730130 A4 | -------------- |

## INTERNATIONAL SEARCH REPORT

| International application No. |
|---|
| PCT/MX2023/050062 |

CLASSIFICATION OF SUBJECT MATTER

*A61K31/07* (2006.01)
*A61K36/45* (2006.01)
*A61K31/201* (2006.01)
*A61K31/202* (2006.01)
*A61P27/02* (2006.01)

Form PCT/ISA/210 (extra sheet) (July 2022)

## REFERENCES CITED IN THE DESCRIPTION

**Patent documents cited in the description**

- WO 2004006801 A

**Non-patent literature cited in the description**

- **DALIA NG ; J.C. ALTAMIRANO-VALLEJO ; A. GONZALEZ-DE LA ROSA et al.** An Oral Polyphenol Formulation to Modulate the Ocular Surface Inflammatory Process and to Improve the Symptomatology Associated with Dry Eye Disease. *Nutrients*, vol. 14 (15), 3236